# EUROPEAN PATENT SPECIFICATION

(11) **EP 4 076 048 B1**
(45) Date of publication and mention of the grant of the patent: **11.10.2023**
(21) Application number: 20829898.4
(22) Date of filing: 16.12.2020
(51) Int. Cl.: A24F 40/00, A61M 15/06, A61M 11/00

(54) **AN AEROSOL-GENERATING ARTICLE COMPRISING A BARRIER**
AEROSOLERZEUGENDER ARTIKEL MIT EINER BARRIERE
ARTICLE DE GÉNÉRATION D'AÉROSOL COMPRENANT UNE BARRIÈRE

(30) Priority: 18.12.2019 EP 19217723
(43) Date of publication of application: 26.10.2022
(73) Proprietor: Philip Morris Products S.A., 2000 Neuchâtel (CH)
(72) Inventor: SPADARO, Fabiana, 2000 Neuchâtel (CH); TAURINO, Irene, 2000 Neuchâtel (CH)
(74) Representative: Whitfield, Ian
(86) International application number: PCT/EP2020/086488
(87) International publication number: WO 2021/122791

(56) References cited:
- WO-A1-2015/070405
- WO-A1-2015/117702
- WO-A1-2015/117703
- WO-A1-2016/118005
- WO-A1-2020/070109
- US-A1- 2015 313 285

## Description

The present invention relates to an aerosol-generating article comprising a barrier. The present invention also relates to an aerosol-generating system comprising an aerosol-generating article comprising a barrier.

Aerosol-generating systems for delivering to a user typically comprise an atomiser configured to generate an inhalable aerosol from an aerosol-forming substrate. Some known aerosol-generating systems comprise a thermal atomiser such as an electric heater that is configured to heat and vaporise the aerosol-forming substrate to generate an aerosol. Typical aerosol-forming substrates for use in aerosol-generating systems are nicotine formulations, which may be liquid nicotine formulations comprising an aerosol former such as glycerine and/or propylene glycol.

WO2015070405A1 discloses an electronic cigarette comprising an oil-storage mechanism and an atomizing component. The oil-storage mechanism is provided with a cigarette oil flowing channel used to output cigarette oil stored within the oil storage mechanism to the atomizing component and with a hot melt sealing structure used for sealing the cigarette oil flowing channel. When the atomizer is powered on for the first time, the hot melt sealing structure is heated and melted to open the cigarette oil flowing channel.

US2015313285A1 discloses an aerosol-generating system including an encapsulated volatile liquid source comprising a sorption element, a volatile liquid sorbed on the sorption element, and a sealant encapsulating the sorption element. The sealant has a melting point of between about 40 degrees Celsius and about 120 degrees Celsius.

It would be desirable to provide an aerosol-generating article that exhibits reduced risk of leakage compared to typical aerosol-generating articles when used in an aerosol-generating system.

There is provided an aerosol-generating article for use in an aerosol-generating system, the aerosol-generating article comprising: a storage portion having an outlet; an aerosol-forming substrate, the aerosol-forming substrate being contained within the storage portion; and a vaporisable barrier configured to seal the outlet, wherein the vaporisable barrier comprises one or more polymers, wherein the vaporisable barrier has a polymer content of greater than or equal to 1 percent by weight, wherein the vaporisable barrier is configured to be vaporised during a first heating cycle of the aerosol-generating article during first use of the aerosol-generating article in the aerosol-generating system, and wherein the vaporisable barrier comprises one or more metal salts, wherein the one or more metal salts are selected from the group consisting of metal alginates, metal benzoates, metal cinnamates, metal cycloheptanecarboxylates, metal levulinates, metal propanoates, metal stearates and metal undecanoates, preferably wherein the vaporisable barrier comprises one or more metal stearates.

There is also provided an aerosol-generating article for use in an aerosol-generating system, the aerosol-generating article comprising: a storage portion; an aerosol-forming substrate, the aerosol-forming substrate being contained within the storage portion; a heater, the heater being configured to heat the aerosol-forming substrate; and a vaporisable barrier configured to seal the storage portion by blocking the flow of the aerosol forming substrate from the storage portion to the heater, the vaporisable barrier being provided between the storage portion and the heater, wherein the vaporisable barrier comprises one or more polymers, wherein the vaporisable barrier has a polymer content of greater than or equal to 1 percent by weight, wherein the vaporisable barrier is configured to be vaporised during a first heating cycle of the aerosol-generating article during first use of the aerosol-generating article in the aerosol-generating system, and wherein the vaporisable barrier comprises one or more metal salts, wherein the one or more metal salts are selected from the group consisting of metal alginates, metal benzoates, metal cinnamates, metal cycloheptanecarboxylates, metal levulinates, metal propanoates, metal stearates and metal undecanoates, preferably wherein the vaporisable barrier comprises one or more metal stearates.

There is also provided a method of manufacturing an aerosol-generating article, the method comprising: providing an aerosol generating article comprising: a storage portion; an aerosol-forming substrate, the aerosol-forming substrate being contained within the storage portion; and a heater, the heater being configured to heat the aerosol-forming substrate; and providing a vaporisable barrier between the storage portion and the heater the vaporisable barrier being configured to seal the storage portion by blocking the flow of the aerosol-forming substrate from the storage portion to the heater, wherein the vaporisable barrier comprises one or more polymers, wherein the vaporisable barrier has a polymer content of greater than or equal to 1 percent by weight, wherein the vaporisable barrier is configured to be vaporised during a first heating cycle of the aerosol-generating article during first use of the aerosol-generating article in the aerosol-generating system, and wherein the vaporisable barrier comprises one or more metal salts, wherein the one or more metal salts are selected from the group consisting of metal alginates, metal benzoates, metal cinnamates, metal cycloheptanecarboxylates, metal levulinates, metal propanoates, metal stearates and metal undecanoates, preferably wherein the vaporisable barrier comprises one or more metal stearates.

As used herein, the term "aerosol-forming substrate" relates to a substrate capable of releasing volatile compounds that can form an aerosol. Such volatile compounds may be released by heating the aerosol-forming substrate or by other aerosolising means. The aerosol-forming substrate may be a liquid. The liquid may be an e-liquid. The liquid may be a solution. The colloid may have discontinuous solid particles dispersed in a continuous liquid. The colloid may have discontinuous liquid particles dispersed in a continuous liquid. The colloid may have discontinuous liquid particles dispersed in a continuous solid.

As used herein, the term "vaporisable" means that substantially all of the mass of the seal enters the vapour phase in the normal heating cycle of the aerosol-generating article, the aerosol-generating device or the aerosol-generating system. That is preferably at least about 95% of the mass of the seal enters the vapour phase in a normal heating cycle of the aerosol-generating article, more preferably at least about 99% of the mass, most preferably all of the mass enters the vapour phase.

Unless stated otherwise, percentages by weight of components of the barrier recited herein are based on the total weight of the barrier.

Advantageously, the vaporisable barrier is a solid impermeable layer that internally seals the storage portion containing the aerosol-forming substrate. Sealing the storage portion may advantageously reduce the risk of leakage of the aerosol-forming substrate from the storage portion before first use, which improves shelf life.

The vaporisable barrier may even reduce or prevent leakage under reduced pressure such as during transportation on an aeroplane.

The vaporisable barrier may be put in place during the manufacturing process, so there is no need for a large window for a sealing tab, which gives more freedom for designing an aerosol-generating article. Not having a sealing tab also simplifies the assembly process.

The solid vaporisable barrier is vaporised by the heater during first use of the aerosol-generating article, aerosol-generating device or aerosol-generating system (that is, during the first heating cycle). This allows the aerosol-generating article, aerosol-generating device or aerosol-generating system to be used as normal by a user.

The vaporisable barrier may be thermally degradable when heated. Preferably, the vaporisable barrier may be thermally degradable when heated to a temperature of between 60 degrees Celsius and 300 degrees Celsius. More preferably, the vaporisable barrier may be thermally degradable when heated to a temperature of between 60 degrees Celsius and 270 degrees Celsius. The vaporisable barrier may be thermally degradable when heated to a temperature of between 180 degrees Celsius and 300 degrees Celsius. The vaporisable barrier may be thermally degradable when heated to a temperature of between 180 degrees Celsius and 270 degrees Celsius.

The vaporisable barrier may comprise a material that is thermally degradable when heated. The vaporisable barrier may comprise a material that is thermally degradable when heated to a temperature of between 60 degrees Celsius and 300 degrees Celsius. The vaporisable barrier may comprise a material that is thermally degradable when heated to a temperature of between 60 degrees Celsius and 270 degrees Celsius. The vaporisable barrier may comprise a material that is thermally degradable when heated to a temperature of between 180 degrees Celsius and 300 degrees Celsius. The vaporisable barrier may comprise a material that is thermally degradable when heated to a temperature of between 180 degrees Celsius and 270 degrees Celsius.

In an example, the vaporisable barrier thermally degrades when it begins to melt when heated.

The vaporisable barrier may comprise a material that is vaporisable when heated to a temperature of between 180 degrees Celsius and 300 degrees Celsius. Preferably, the vaporisable barrier may comprise a material that is vaporisable when heated to a temperature of between 180 degrees Celsius and 270 degrees Celsius.

The vaporisable barrier may be vaporisable when heated to a temperature of between 180 degrees Celsius and 300 degrees Celsius. Preferably, the vaporisable barrier may be vaporisable when heated to a temperature of between 180 degrees Celsius and 270 degrees Celsius.

The vaporisable barrier may have a polymer content of greater than or equal to 2 percent by weight. The vaporisable barrier may have a polymer content of greater than or equal to 3 percent by weight. The vaporisable barrier may have a polymer content of greater than or equal to 4 percent by weight.

The vaporisable barrier may have a polymer content of greater than or equal to 5 percent by weight. Advantageously, a polymer content of greater than or equal to 5 percent by weight provides a reliable seal. The vaporisable barrier may have a polymer content of greater than or equal to 8 percent by weight. The vaporisable barrier may have a polymer content of greater than or equal to 10 percent by weight. The vaporisable barrier may have a polymer content of greater than or equal to 15 percent by weight. The vaporisable barrier may have a polymer content of greater than or equal to 20 percent by weight. The vaporisable barrier may have a polymer content of greater than or equal to 25 percent by weight. The vaporisable barrier may have a polymer content of greater than or equal to 30 percent by weight. The vaporisable barrier may have a polymer content of greater than or equal to 35 percent by weight.

The vaporisable barrier may have a polymer content of less than or equal to 50 percent by weight. The vaporisable barrier may have a polymer content of less than or equal to 45 percent by weight. The vaporisable barrier may have a polymer content of less than or equal to 40 percent by weight. The vaporisable barrier may have a polymer content of less than or equal to 35 percent by weight. The vaporisable barrier may have a polymer content of less than or equal to 30 percent by weight. The vaporisable barrier may have a polymer content of less than or equal to 25 percent by weight. The vaporisable barrier may have a polymer content of less than or equal to 20 percent by weight. The vaporisable barrier may have a polymer content of less than or equal to 15 percent by weight. The vaporisable barrier may have a polymer content of less than or equal to 10 percent by weight. The vaporisable barrier may have a polymer content of less than or equal to 8 percent by weight. The vaporisable barrier may have a polymer content of less than or equal to 5 percent by weight.

The vaporisable barrier may have a polymer content of between about 1 percent by weight and about 40 percent by weight. The vaporisable barrier may have a polymer content of between about 2 percent by weight and about 40 percent by weight. The vaporisable barrier may have a polymer content of between about 3 percent by weight and about 40 percent by weight. The vaporisable barrier may have a polymer content of between about 4 percent by weight and about 40 percent by weight. The vaporisable barrier may have a polymer content of between about 5 percent by weight and about 40 percent by weight. The vaporisable barrier may have a polymer content of between about 8 percent by weight and about 40 percent by weight. The vaporisable barrier may have a polymer content of between about 10 percent by weight and about 40 percent by weight. The vaporisable barrier may have a polymer content of between about 15 percent by weight and about 40 percent by weight. The vaporisable barrier may have a polymer content of between about 20 percent by weight and about 40 percent by weight. The vaporisable barrier may have a polymer content of between about 25 percent by weight and about 40 percent by weight. The vaporisable barrier may have a polymer content of between about 30 percent by weight and about 40 percent by weight. The vaporisable barrier may have a polymer content of between about 35 percent by weight and about 40 percent by weight.

The vaporisable barrier may have a polymer content of between about 5 percent by weight and about 40 percent by weight. The vaporisable barrier may have a polymer content of between about 5 percent by weight and about 35 percent by weight. The vaporisable barrier may have a polymer content of between about 5 percent by weight and about 30 percent by weight. The vaporisable barrier may have a polymer content of between about 5 percent by weight and about 25 percent by weight. The vaporisable barrier may have a polymer content of between about 5 percent by weight and about 20 percent by weight. The vaporisable barrier may have a polymer content of between about 5 percent by weight and about 15 percent by weight. The vaporisable barrier may have a polymer content of between about 5 percent by weight and about 10 percent by weight. The vaporisable barrier may have a polymer content of between about 5 percent by weight and about 8 percent by weight.

The vaporisable barrier may have a polymer content of between about 8 percent by weight and about 35 percent by weight. The vaporisable barrier may have a polymer content of between about 10 percent by weight and about 35 percent by weight. The vaporisable barrier may have a polymer content of between about 10 percent by weight and about 30 percent by weight. The vaporisable barrier may have a polymer content of between about 15 percent by weight and about 30 percent by weight. The vaporisable barrier may have a polymer content of between about 15 percent by weight and about 25 percent by weight. The vaporisable barrier may have a polymer content of between about 20 percent by weight and about 25 percent by weight.

The vaporisable barrier may have a polymer content of between about 10 percent by weight and about 15 percent by weight.

The vaporisable barrier may be formed from a formulation comprising one or more polymers.

The formulation may have a polymer content of greater than or equal to 1 percent by weight. The formulation may have a polymer content of greater than or equal to 2 percent by weight. The formulation may have a polymer content of greater than or equal to 3 percent by weight. The formulation may have a polymer content of greater than or equal to 4 percent by weight.

The formulation may have a polymer content of greater than or equal to 5 percent by weight. Advantageously, a polymer content of greater than or equal to 5 percent by weight provides a reliable seal. The formulation may have a polymer content of greater than or equal to 8 percent by weight. The formulation may have a polymer content of greater than or equal to 10 percent by weight. The formulation may have a polymer content of greater than or equal to 15 percent by weight.

The formulation may have a polymer content of less than or equal to 20 percent by weight. The formulation may have a polymer content of less than or equal to 15 percent by weight. The formulation may have a polymer content of less than or equal to 10 percent by weight. The formulation may have a polymer content of less than or equal to 8 percent by weight. The formulation may have a polymer content of less than or equal to 5 percent by weight. The formulation may have a polymer content of less than or equal to 4 percent by weight. The formulation may have a polymer content of less than or equal to 3 percent by weight. The formulation may have a polymer content of less than or equal to 2 percent by weight.

The formulation may have a polymer content of between about 1 percent by weight and about 20 percent by weight. The formulation may have a polymer content of between about 2 percent by weight and about 20 percent by weight. The formulation may have a polymer content of between about 3 percent by weight and about 20 percent by weight. The formulation may have a polymer content of between about 4 percent by weight and about 20 percent by weight. The formulation may have a polymer content of between about 5 percent by weight and about 20 percent by weight. The formulation may have a polymer content of between about 8 percent by weight and about 20 percent by weight. The formulation may have a polymer content of between about 10 percent by weight and about 20 percent by weight. The formulation may have a polymer content of between about 15 percent by weight and about 20 percent by weight.

The formulation may have a polymer content of between about 1 percent by weight and about 20 percent by weight. The formulation may have a polymer content of between about 1 percent by weight and about 15 percent by weight. The formulation may have a polymer content of between about 1 percent by weight and about 10 percent by weight. The formulation may have a polymer content of between about 1 percent by weight and about 8 percent by weight. The formulation may have a polymer content of between about 1 percent by weight and about 5 percent by weight. The formulation may have a polymer content of between about 1 percent by weight and about 4 percent by weight. The formulation may have a polymer content of between about 1 percent by weight and about 3 percent by weight. The formulation may have a polymer content of between about 1 percent by weight and about 2 percent by weight.

The formulation may have a polymer content of between about 5 percent by weight and about 15 percent by weight. The formulation may have a polymer content of between about 5 percent by weight and about 10 percent by weight. The formulation may have a polymer content of between about 5 percent by weight and about 8 percent by weight.

The formulation may have a polymer content of between about 8 percent by weight and about 15 percent by weight. The formulation may have a polymer content of between about 8 percent by weight and about 10 percent by weight.

The formulation may have a polymer content of between about 10 percent by weight and about 15 percent by weight.

The one or more polymers may be selected from the group consisting of: polyvinyl acetate, polyvinyl alcohol, polyethylene glycol, polyglycolic acid, polylactic acid, polydioxanone, polycaprolactone, polyethylene, polypropylene glycol and starch.

The polyethylene may be low density polyethylene.

Preferably, the one or more polymers are selected from the group consisting of: polyvinyl alcohol, polyethylene glycol, polypropylene glycol and starch.

More preferably, the one or more polymers are selected from the group consisting of: polyvinyl alcohol, polyethylene glycol and polypropylene glycol.

Even more preferably, the one or more polymers are selected from the group consisting of: polyvinyl alcohol and polyethylene glycol.

Most preferably, the one or more polymers consists of polyvinyl alcohol.

All starches are made up from varying proportions of amylose and amylopectin. The selection of the particular starch for the vaporisable barrier or formulation may be based on the ratio of amylose to amylopectin, which depends on the desired function of the starch. The starch may be corn starch or wheat starch. Preferably, the starch is corn starch, preferably the starch is waxy corn starch. Waxy corn starch is essentially pure amylopectin containing only trace amounts of amylose. Waxy corn starches have been found to produce a better, more elastic barrier. It should be noted that the weight average molecular weight of the starch varies due to natural variation.

The one or more polymers may have a weight average molecular weight of greater than or equal to 6000 g/mol. The one or more polymers may have a weight average molecular weight of greater than or equal to 60000 g/mol. The one or more polymers may have a weight average molecular weight of greater than or equal to 100000 g/mol. The one or more polymers may have a weight average molecular weight of greater than or equal to 140000 g/mol. The one or more polymers may have a weight average molecular weight of greater than or equal to 200000 g/mol.

The one or more polymers may have a weight average molecular weight (M_{w}) of less than or equal to 8000000 g/mol. The one or more polymers may have a weight average molecular weight of less than or equal to 5000000 g/mol. The one or more polymers may have a weight average molecular weight of less than or equal to 2000000 g/mol. The one or more polymers may have a weight average molecular weight of less than or equal to 1000000 g/mol. The one or more polymers may have a weight average molecular weight of less than or equal to 500000 g/mol. The one or more polymers may have a weight average molecular weight of less than or equal to 200000 g/mol. The one or more polymers may have a weight average molecular weight of less than or equal to 190000 g/mol.

The one or more polymers may have a weight average molecular weight of between 6000 g/mol and 8000000 g/mol. The one or more polymers may have a weight average molecular weight of between 60000 g/mol and 500000 g/mol. The one or more polymers may have a weight average molecular weight of between 100000 g/mol and 200000 g/mol. The one or more polymers may have a weight average molecular weight of between 140000 g/mol and 190000 g/mol.

The vaporisable barrier may have a metal salt content of greater than or equal to about 0.1 percent by weight. The vaporisable barrier may have a metal salt content of greater than or equal to about 0.25 percent by weight. The vaporisable barrier may have a metal salt content of greater than or equal to about 0.5 percent by weight. The vaporisable barrier may have a metal salt content of greater than or equal to about 0.75 percent by weight. The vaporisable barrier may have a metal salt content of greater than or equal to about 1 percent by weight. The vaporisable barrier may have a metal salt content of greater than or equal to about 1.25 percent by weight. The vaporisable barrier may have a metal salt content of greater than or equal to about 1.5 percent by weight. The vaporisable barrier may have a metal salt content of greater than or equal to about 1.75 percent by weight. The vaporisable barrier may have a metal salt content of greater than or equal to about 2 percent by weight. The vaporisable barrier may have a metal salt content of greater than or equal to about 3 percent by weight. The vaporisable barrier may have a metal salt content of greater than or equal to about 4 percent by weight. The vaporisable barrier may have a metal salt content of greater than or equal to about 5 percent by weight. The vaporisable barrier may have a metal salt content of greater than or equal to about 6 percent by weight. The vaporisable barrier may have a metal salt content of greater than or equal to about 7 percent by weight. The vaporisable barrier may have a metal salt content of greater than or equal to about 8 percent by weight. The vaporisable barrier may have a metal salt content of greater than or equal to about 9 percent by weight.

The vaporisable barrier may have a metal salt content of less than or equal to about 15 percent by weight. The vaporisable barrier may have a metal salt content of less than or equal to about 12 percent by weight. The vaporisable barrier may have a metal salt content of less than or equal to about 10 percent by weight. The vaporisable barrier may have a metal salt content of less than or equal to about 9 percent by weight. The vaporisable barrier may have a metal salt content of less than or equal to about 8 percent by weight. The vaporisable barrier may have a metal salt content of less than or equal to about 7 percent by weight. The vaporisable barrier may have a metal salt content of less than or equal to about 6 percent by weight. The vaporisable barrier may have a metal salt content of less than or equal to about 5 percent by weight. The vaporisable barrier may have a metal salt content of less than or equal to about 4 percent by weight. The vaporisable barrier may have a metal salt content of less than or equal to about 3 percent by weight. The vaporisable barrier may have a metal salt content of less than or equal to about 2 percent by weight. The vaporisable barrier may have a metal salt content of less than or equal to about 1.75 percent by weight. The vaporisable barrier may have a metal salt content of less than or equal to about 1.5 percent by weight. The vaporisable barrier may have a metal salt content of less than or equal to about 1.25 percent by weight. The vaporisable barrier may have a metal salt content of less than or equal to about 1 percent by weight. The vaporisable barrier may have a metal salt content of less than or equal to about 0.75 percent by weight. The vaporisable barrier may have a metal salt content of less than or equal to about 0.5 percent by weight. The vaporisable barrier may have a metal salt content of less than or equal to about 0.25 percent by weight.

The vaporisable barrier may have a metal salt content of between about 0.1 percent by weight and about 10 percent by weight. The vaporisable barrier may have a metal salt content of between about 0.1 percent by weight and about 9 percent by weight. The vaporisable barrier may have a metal salt content of between about 0.1 percent by weight and about 8 percent by weight. The vaporisable barrier may have a metal salt content of between about 0.1 percent by weight and about 7 percent by weight. The vaporisable barrier may have a metal salt content of between about 0.1 percent by weight and about 6 percent by weight. The vaporisable barrier may have a metal salt content of between about 0.1 percent by weight and about 5 percent by weight. The vaporisable barrier may have a metal salt content of between about 0.1 percent by weight and about 4 percent by weight. The vaporisable barrier may have a metal salt content of between about 0.1 percent by weight and about 3 percent by weight. The vaporisable barrier may have a metal salt content of between about 0.1 percent by weight and about 2 percent by weight. The vaporisable barrier may have a metal salt content of between about 0.1 percent by weight and about 1.75 percent by weight. The vaporisable barrier may have a metal salt content of between about 0.1 percent by weight and about 1.5 percent by weight. The vaporisable barrier may have a metal salt content of between about 0.1 percent by weight and about 1.25 percent by weight. The vaporisable barrier may have a metal salt content of between about 0.1 percent by weight and about 1 percent by weight. The vaporisable barrier may have a metal salt content of between about 0.1 percent by weight and about 0.75 percent by weight.

The vaporisable barrier may have a metal salt content of between about 0.25 percent by weight and about 10 percent by weight. The vaporisable barrier may have a metal salt content of between about 0.5 percent by weight and about 10 percent by weight. The vaporisable barrier may have a metal salt content of between about 0.75 percent by weight and about 10 percent by weight. The vaporisable barrier may have a metal salt content of between about 1 percent by weight and about 10 percent by weight. The vaporisable barrier may have a metal salt content of between about 1.25 percent by weight and about 10 percent by weight. The vaporisable barrier may have a metal salt content of between about 1.5 percent by weight and about 10 percent by weight. The vaporisable barrier may have a metal salt content of between about 1.75 percent by weight and about 10 percent by weight. The vaporisable barrier may have a metal salt content of between about 2 percent by weight and about 10 percent by weight. The vaporisable barrier may have a metal salt content of between about 3 percent by weight and about 10 percent by weight. The vaporisable barrier may have a metal salt content of between about 4 percent by weight and about 10 percent by weight. The vaporisable barrier may have a metal salt content of between about 5 percent by weight and about 10 percent by weight. The vaporisable barrier may have a metal salt content of between about 6 percent by weight and about 10 percent by weight. The vaporisable barrier may have a metal salt content of between about 7 percent by weight and about 10 percent by weight. The vaporisable barrier may have a metal salt content of between about 8 percent by weight and about 10 percent by weight. The vaporisable barrier may have a metal salt content of between about 9 percent by weight and about 10 percent by weight.

The vaporisable barrier may have a metal salt content of between about 0.25 percent by weight and about 9 percent by weight. The vaporisable barrier may have a metal salt content of between about 0.5 percent by weight and about 9 percent by weight. The vaporisable barrier may have a metal salt content of between about 0.5 percent by weight and about 8 percent by weight. The vaporisable barrier may have a metal salt content of between about 0.75 percent by weight and about 8 percent by weight. The vaporisable barrier may have a metal salt content of between about 0.75 percent by weight and about 7 percent by weight. The vaporisable barrier may have a metal salt content of between about 1 percent by weight and about 7 percent by weight. The vaporisable barrier may have a metal salt content of between about 1 percent by weight and about 6 percent by weight. The vaporisable barrier may have a metal salt content of between about 1.25 percent by weight and about 6 percent by weight. The vaporisable barrier may have a metal salt content of between about 1.25 percent by weight and about 5 percent by weight. The vaporisable barrier may have a metal salt content of between about 1.5 percent by weight and about 5 percent by weight. The vaporisable barrier may have a metal salt content of between about 1.5 percent by weight and about 4 percent by weight. The vaporisable barrier may have a metal salt content of between about 1.75 percent by weight and about 4 percent by weight. The vaporisable barrier may have a metal salt content of between about 1.75 percent by weight and about 3 percent by weight. The vaporisable barrier may have a metal salt content of between about 2 percent by weight and about 3 percent by weight.

The vaporisable barrier may be formed from a formulation comprising one or more metal salts.

The formulation may have a metal salt content of greater than or equal to about 0.1 percent by weight. The formulation may have a metal salt content of greater than or equal to about 0.25 percent by weight. The formulation may have a metal salt content of greater than or equal to about 0.5 percent by weight. The formulation may have a metal salt content of greater than or equal to about 0.75 percent by weight. The formulation may have a metal salt content of greater than or equal to about 1 percent by weight. The formulation may have a metal salt content of greater than or equal to about 1.25 percent by weight. The formulation may have a metal salt content of greater than or equal to about 1.5 percent by weight. The formulation may have a metal salt content of greater than or equal to about 1.75 percent by weight. The formulation may have a metal salt content of greater than or equal to about 2 percent by weight. The formulation may have a metal salt content of greater than or equal to about 3 percent by weight. The formulation may have a metal salt content of greater than or equal to about 4 percent by weight. The formulation may have a metal salt content of greater than or equal to about 5 percent by weight.

The formulation may have a metal salt content of less than or equal to about 10 percent by weight. The formulation may have a metal salt content of less than or equal to about 5 percent by weight. The formulation may have a metal salt content of less than or equal to about 4 percent by weight. The formulation may have a metal salt content of less than or equal to about 3 percent by weight. The formulation may have a metal salt content of less than or equal to about 2 percent by weight. The formulation may have a metal salt content of less than or equal to about 1.75 percent by weight. The formulation may have a metal salt content of less than or equal to about 1.5 percent by weight. The formulation may have a metal salt content of less than or equal to about 1.25 percent by weight. The formulation may have a metal salt content of less than or equal to about 1 percent by weight. The formulation may have a metal salt content of less than or equal to about 0.75 percent by weight. The formulation may have a metal salt content of less than or equal to about 0.5 percent by weight. The formulation may have a metal salt content of less than or equal to about 0.25 percent by weight.

The formulation may have a metal salt content of between about 0.1 percent by weight and about 10 percent by weight. The formulation may have a metal salt content of between about 0.1 percent by weight and about 5 percent by weight. The formulation may have a metal salt content of between about 0.1 percent by weight and about 4 percent by weight. The formulation may have a metal salt content of between about 0.1 percent by weight and about 3 percent by weight. The formulation may have a metal salt content of between about 0.1 percent by weight and about 2 percent by weight. The formulation may have a metal salt content of between about 0.1 percent by weight and about 1.75 percent by weight. The formulation may have a metal salt content of between about 0.1 percent by weight and about 1.5 percent by weight. The formulation may have a metal salt content of between about 0.1 percent by weight and about 1.25 percent by weight. The formulation may have a metal salt content of between about 0.1 percent by weight and about 1 percent by weight. The formulation may have a metal salt content of between about 0.1 percent by weight and about 0.75 percent by weight.

The formulation may have a metal salt content of between about 0.1 percent by weight and about 2 percent by weight. The formulation may have a metal salt content of between about 0.25 percent by weight and about 2 percent by weight. The formulation may have a metal salt content of between about 0.5 percent by weight and about 2 percent by weight. The formulation may have a metal salt content of between about 0.75 percent by weight and about 2 percent by weight. The formulation may have a metal salt content of between about 1 percent by weight and about 2 percent by weight. The formulation may have a metal salt content of between about 1.25 percent by weight and about 2 percent by weight. The formulation may have a metal salt content of between about 1.5 percent by weight and about 2 percent by weight. The formulation may have a metal salt content of between about 1.75 percent by weight and about 2 percent by weight.

The one or more metal salts may be selected from the group consisting of metal benzoates, metal cinnamates, metal cycloheptanecarboxylates, metal levulinates, metal propanoates, metal stearates and metal undecanoates.

The one or more metal salts may be selected from the group consisting of metal cinnamates, metal cycloheptanecarboxylates, metal levulinates, metal propanoates, metal stearates and metal undecanoates.

The one or more salts may be salts of any suitable metal.

Preferably, the one or more metal salts are alkali metal salts.

More preferably, the one or more metal salts are sodium salts.

Preferably, the one or more metal salts are non-saccharide sodium salts.

More preferably, the one or more sodium salts are selected from the group consisting of sodium benzoate, sodium cinnamate, sodium cycloheptanecarboxylate, sodium levulinate, sodium propanoate, sodium stearate and sodium undecanoate.

Most preferably, the one or more metal salts is sodium stearate.

Advantageously, when the vaporisable barrier contains sodium stearate, there is increased hydrophobicity of the seal, which leads to decreased permeability of the aerosol-forming substrate, thereby resulting in increased shelf life of the aerosol-generating article, the aerosol-generating device or the aerosol-generating system.

The vaporisable barrier may comprise one or more plasticizers.

The vaporisable barrier may have a plasticizer content of greater than or equal to about 0.1 percent by weight. The vaporisable barrier may have a plasticizer content of greater than or equal to about 0.5 percent by weight. The vaporisable barrier may have a plasticizer content of greater than or equal to about 1 percent by weight. The vaporisable barrier may have a plasticizer content of greater than or equal to about 2 percent by weight. The vaporisable barrier may have a plasticizer content of greater than or equal to about 3 percent by weight. The vaporisable barrier may have a plasticizer content of greater than or equal to about 4 percent by weight. The vaporisable barrier may have a plasticizer content of greater than or equal to about 5 percent by weight. The vaporisable barrier may have a plasticizer content of greater than or equal to about 10 percent by weight. The vaporisable barrier may have a plasticizer content of greater than or equal to about 15 percent by weight. The vaporisable barrier may have a plasticizer content of greater than or equal to about 20 percent by weight. The vaporisable barrier may have a plasticizer content of greater than or equal to about 25 percent by weight. The vaporisable barrier may have a plasticizer content of greater than or equal to about 30 percent by weight. The vaporisable barrier may have a plasticizer content of greater than or equal to about 35 percent by weight. The vaporisable barrier may have a plasticizer content of greater than or equal to about 40 percent by weight. The vaporisable barrier may have a plasticizer content of greater than or equal to about 45 percent by weight. The vaporisable barrier may have a plasticizer content of greater than or equal to about 50 percent by weight. The vaporisable barrier may have a plasticizer content of greater than or equal to about 55 percent by weight. The vaporisable barrier may have a plasticizer content of greater than or equal to about 60 percent by weight. The vaporisable barrier may have a plasticizer content of greater than or equal to about 65 percent by weight. The vaporisable barrier may have a plasticizer content of greater than or equal to about 70 percent by weight. The vaporisable barrier may have a plasticizer content of greater than or equal to about 75 percent by weight. The vaporisable barrier may have a plasticizer content of greater than or equal to about 80 percent by weight. The vaporisable barrier may have a plasticizer content of greater than or equal to about 85 percent by weight.

The vaporisable barrier may have a plasticizer content of less than or equal to about 90 percent by weight. The vaporisable barrier may have a plasticizer content of less than or equal to about 85 percent by weight. The vaporisable barrier may have a plasticizer content of less than or equal to about 80 percent by weight. The vaporisable barrier may have a plasticizer content of less than or equal to about 75 percent by weight. The vaporisable barrier may have a plasticizer content of less than or equal to about 70 percent by weight. The vaporisable barrier may have a plasticizer content of less than or equal to about 65 percent by weight. The vaporisable barrier may have a plasticizer content of less than or equal to about 60 percent by weight. The vaporisable barrier may have a plasticizer content of less than or equal to about 55 percent by weight. The vaporisable barrier may have a plasticizer content of less than or equal to about 50 percent by weight. The vaporisable barrier may have a plasticizer content of less than or equal to about 45 percent by weight. The vaporisable barrier may have a plasticizer content of less than or equal to about 40 percent by weight. The vaporisable barrier may have a plasticizer content of less than or equal to about 35 percent by weight. The vaporisable barrier may have a plasticizer content of less than or equal to about 30 percent by weight. The vaporisable barrier may have a plasticizer content of less than or equal to about 25 percent by weight. The vaporisable barrier may have a plasticizer content of less than or equal to about 20 percent by weight. The vaporisable barrier may have a plasticizer content of less than or equal to about 15 percent by weight. The vaporisable barrier may have a plasticizer content of less than or equal to about 10 percent by weight. The vaporisable barrier may have a plasticizer content of less than or equal to about 5 percent by weight. The vaporisable barrier may have a plasticizer content of less than or equal to about 4 percent by weight. The vaporisable barrier may have a plasticizer content of less than or equal to about 3 percent by weight.

The vaporisable barrier may have a plasticizer content of between about 2 percent and about 90 percent by weight. The vaporisable barrier may have a plasticizer content of between about 2 percent and about 85 percent by weight. The vaporisable barrier may have a plasticizer content of between about 2 percent and about 80 percent by weight. The vaporisable barrier may have a plasticizer content of between about 2 percent and about 75 percent by weight. The vaporisable barrier may have a plasticizer content of between about 2 percent and about 70 percent by weight. The vaporisable barrier may have a plasticizer content of between about 2 percent and about 65 percent by weight. The vaporisable barrier may have a plasticizer content of between about 2 percent and about 60 percent by weight. The vaporisable barrier may have a plasticizer content of between about 2 percent and about 55 percent by weight. The vaporisable barrier may have a plasticizer content of between about 2 percent and about 50 percent by weight. The vaporisable barrier may have a plasticizer content of between about 2 percent and about 45 percent by weight. The vaporisable barrier may have a plasticizer content of between about 2 percent and about 40 percent by weight. The vaporisable barrier may have a plasticizer content of between about 2 percent and about 35 percent by weight. The vaporisable barrier may have a plasticizer content of between about 2 percent and about 30 percent by weight. The vaporisable barrier may have a plasticizer content of between about 2 percent and about 25 percent by weight. The vaporisable barrier may have a plasticizer content of between about 2 percent and about 20 percent by weight. The vaporisable barrier may have a plasticizer content of between about 2 percent and about 15 percent by weight. The vaporisable barrier may have a plasticizer content of between about 2 percent and about 10 percent by weight. The vaporisable barrier may have a plasticizer content of between about 2 percent and about 5 percent by weight. The vaporisable barrier may have a plasticizer content of between about 2 percent and about 4 percent by weight. The vaporisable barrier may have a plasticizer content of between about 2 percent and about 3 percent by weight.

The vaporisable barrier may have a plasticizer content of between about 1 percent and about 90 percent by weight. The vaporisable barrier may have a plasticizer content of between about 3 percent and about 90 percent by weight. The vaporisable barrier may have a plasticizer content of between about 4 percent and about 90 percent by weight. The vaporisable barrier may have a plasticizer content of between about 5 percent and about 90 percent by weight. The vaporisable barrier may have a plasticizer content of between about 10 percent and about 90 percent by weight. The vaporisable barrier may have a plasticizer content of between about 15 percent and about 90 percent by weight. The vaporisable barrier may have a plasticizer content of between about 20 percent and about 90 percent by weight. The vaporisable barrier may have a plasticizer content of between about 25 percent and about 90 percent by weight. The vaporisable barrier may have a plasticizer content of between about 30 percent and about 90 percent by weight. The vaporisable barrier may have a plasticizer content of between about 35 percent and about 90 percent by weight. The vaporisable barrier may have a plasticizer content of between about 40 percent and about 90 percent by weight. The vaporisable barrier may have a plasticizer content of between about 45 percent and about 90 percent by weight. The vaporisable barrier may have a plasticizer content of between about 50 percent and about 90 percent by weight. The vaporisable barrier may have a plasticizer content of between about 55 percent and about 90 percent by weight. The vaporisable barrier may have a plasticizer content of between about 60 percent and about 90 percent by weight. The vaporisable barrier may have a plasticizer content of between about 65 percent and about 90 percent by weight. The vaporisable barrier may have a plasticizer content of between about 70 percent and about 90 percent by weight. The vaporisable barrier may have a plasticizer content of between about 75 percent and about 90 percent by weight. The vaporisable barrier may have a plasticizer content of between about 80 percent and about 90 percent by weight. The vaporisable barrier may have a plasticizer content of between about 85 percent and about 90 percent by weight.

The vaporisable barrier may have a plasticizer content of between about 3 percent and about 85 percent by weight. The vaporisable barrier may have a plasticizer content of between about 4 percent and about 85 percent by weight. The vaporisable barrier may have a plasticizer content of between about 4 percent and about 80 percent by weight. The vaporisable barrier may have a plasticizer content of between about 5 percent and about 80 percent by weight. The vaporisable barrier may have a plasticizer content of between about 5 percent and about 75 percent by weight. The vaporisable barrier may have a plasticizer content of between about 10 percent and about 70 percent by weight. The vaporisable barrier may have a plasticizer content of between about 10 percent and about 65 percent by weight. The vaporisable barrier may have a plasticizer content of between about 15 percent and about 65 percent by weight. The vaporisable barrier may have a plasticizer content of between about 15 percent and about 60 percent by weight. The vaporisable barrier may have a plasticizer content of between about 20 percent and about 60 percent by weight. The vaporisable barrier may have a plasticizer content of between about 20 percent and about 55 percent by weight. The vaporisable barrier may have a plasticizer content of between about 25 percent and about 55 percent by weight. The vaporisable barrier may have a plasticizer content of between about 25 percent and about 50 percent by weight. The vaporisable barrier may have a plasticizer content of between about 30 percent and about 50 percent by weight. The vaporisable barrier may have a plasticizer content of between about 30 percent and about 45 percent by weight. The vaporisable barrier may have a plasticizer content of between about 35 percent and about 45 percent by weight. The vaporisable barrier may have a plasticizer content of between about 35 percent and about 40 percent by weight.

The vaporisable barrier may be formed from a formulation comprising one or more plasticizers.

The formulation may have a plasticizer content of greater than or equal to about 0.1 percent by weight. The formulation may have a plasticizer content of greater than or equal to about 0.5 percent by weight. The formulation may have a plasticizer content of greater than or equal to about 1 percent by weight. The formulation may have a plasticizer content of greater than or equal to about 2 percent by weight. The formulation may have a plasticizer content of greater than or equal to about 3 percent by weight. The formulation may have a plasticizer content of greater than or equal to about 4 percent by weight. The formulation may have a plasticizer content of greater than or equal to about 5 percent by weight. The formulation may have a plasticizer content of greater than or equal to about 10 percent by weight. The formulation may have a plasticizer content of greater than or equal to about 15 percent by weight. The formulation may have a plasticizer content of greater than or equal to about 20 percent by weight. The formulation may have a plasticizer content of greater than or equal to about 25 percent by weight. The formulation may have a plasticizer content of greater than or equal to about 30 percent by weight. The formulation may have a plasticizer content of greater than or equal to about 35 percent by weight.

The formulation may have a plasticizer content of less than or equal to about 50 percent by weight. The formulation may have a plasticizer content of less than or equal to about 45 percent by weight. The formulation may have a plasticizer content of less than or equal to about 40 percent by weight. The formulation may have a plasticizer content of less than or equal to about 35 percent by weight. The formulation may have a plasticizer content of less than or equal to about 30 percent by weight. The formulation may have a plasticizer content of less than or equal to about 25 percent by weight. The formulation may have a plasticizer content of less than or equal to about 20 percent by weight. The formulation may have a plasticizer content of less than or equal to about 15 percent by weight. The formulation may have a plasticizer content of less than or equal to about 10 percent by weight. The formulation may have a plasticizer content of less than or equal to about 5 percent by weight. The formulation may have a plasticizer content of less than or equal to about 4 percent by weight. The formulation may have a plasticizer content of less than or equal to about 3 percent by weight.

The formulation may have a plasticizer content of between about 2 percent and about 50 percent by weight. The formulation may have a plasticizer content of between about 2 percent and about 45 percent by weight. The formulation may have a plasticizer content of between about 2 percent and about 40 percent by weight. The formulation may have a plasticizer content of between about 2 percent and about 35 percent by weight. The formulation may have a plasticizer content of between about 2 percent and about 30 percent by weight. The formulation may have a plasticizer content of between about 2 percent and about 25 percent by weight. The formulation may have a plasticizer content of between about 2 percent and about 20 percent by weight. The formulation may have a plasticizer content of between about 2 percent and about 15 percent by weight. The formulation may have a plasticizer content of between about 2 percent and about 10 percent by weight. The formulation may have a plasticizer content of between about 2 percent and about 5 percent by weight. The formulation may have a plasticizer content of between about 2 percent and about 4 percent by weight. The formulation may have a plasticizer content of between about 2 percent and about 3 percent by weight.

The formulation may have a plasticizer content of between about 1 percent and about 35 percent by weight. The formulation may have a plasticizer content of between about 3 percent and about 35 percent by weight. The formulation may have a plasticizer content of between about 4 percent and about 35 percent by weight. The formulation may have a plasticizer content of between about 5 percent and about 35 percent by weight. The formulation may have a plasticizer content of between about 10 percent and about 35 percent by weight. The formulation may have a plasticizer content of between about 15 percent and about 35 percent by weight. The formulation may have a plasticizer content of between about 20 percent and about 35 percent by weight. The formulation may have a plasticizer content of between about 25 percent and about 35 percent by weight. The formulation may have a plasticizer content of between about 30 percent and about 35 percent by weight.

Advantageously, including one or more plasticizers in the barrier makes the barrier more stretchable or less rigid or brittle.

The one or more plasticizers may comprise one or more carboxylic acids.

Preferably, the one or more plasticizers comprise one or more carboxylic acids. Advantageously, including one or more carboxylic acids may improve cross-linking within the vaporisable barrier.

The one or more plasticizers may comprise citric acid, lactic acid, tartaric acid, oxalic acid, maleic acid or a combination thereof.

Advantageously, including one or more carboxylic acids in the vaporisable barrier may result in a barrier that is less hygroscopic, which may result in the vaporisable barrier absorbing less water from the environment.

Advantageously, including a plasticizer that contains multiple functional groups, such as a polycarboxylic acid or a carboxylic acid containing additional functional groups, such as lactic acid, may improve cross-linking within the vaporisable barrier.

Advantageously, including a carboxylic acid in the barrier may reduce the amount of aerosol former that is required.

The one or more plasticizers may comprise citric acid. The one or more plasticizers may comprise lactic acid. The one or more plasticizers may comprise tartaric acid. The one or more plasticizers may comprise oxalic acid. The one or more plasticizers may comprise maleic acid.

The one or more plasticizers may comprise one or more aerosol formers.

The one or more aerosol formers may comprise one or more polyhydric alcohols. The one or more polyhydric alcohols may comprise one or more water-miscible polyhydric alcohols. As used herein, the term "water-miscible polyhydric alcohol" describes a polyhydric alcohol that is liquid at 20°C and mixes with water in all proportions to form a homogenous solution.

The one or more aerosol formers may be selected from the group consisting of 1,3-butanediol, glycerine, propylene glycol, triethylene glycol and sorbitol. The glycerine may comprise vegetable glycerine. The glycerine may be synthetic glycerine.

Preferably, the aerosol former is sorbitol. Advantageously, sorbitol is less hygroscopic compared to other aerosol formers, which may result in the vaporisable barrier absorbing less water from the environment and being more stable in humid conditions.

Preferably, the aerosol former is glycerine. Advantageously, glycerine is less volatile compared to other aerosol formers and particularly propylene glycol, which may result in the vaporisable barrier absorbing less water from the environment and being more stable in humid conditions.

Preferably, the aerosol former is propylene glycol. Advantageously, the inclusion of propylene glycol improves the flexibility of the barrier and may improve the vaporisation efficiency of the barrier.

The one or more aerosol formers may comprise a combination of sorbitol and glycerine. The one or more aerosol formers may comprise a combination of sorbitol and propylene glycol. The one or more aerosol formers may comprise a combination of sorbitol, glycerine and propylene glycol. Although sorbitol may improve hygroscopic properties of the vaporisable barrier, including too much sorbitol in the barrier may result in a relatively inflexible barrier that might be less able to cope with pressure changes or imperfections in a material to which it is connected. Adding another aerosol former, or a carboxylic acid, into the barrier may mitigate this problem.

Advantageously, both glycerine and sorbitol may be less volatile than propylene glycol, which may evaporate in warm conditions. The relative amounts of different aerosol former in the barrier may be tailored in order to provide a barrier that is suitable for its intended use and the ambient conditions.

The ratio of the weight percent glycerine content to the weight percent propylene glycol content of the vaporisable barrier may be greater than or equal to about 1. The vaporisable barrier having a higher proportion of glycerine to propylene glycol may result in the vaporisable barrier being harder than with a vaporisable barrier having a higher proportion of propylene glycol to glycerine. Providing a harder vaporisable barrier may advantageously improve sealing.

The ratio of the weight percent glycerine content to the weight percent propylene glycol content of the vaporisable barrier may be greater than or equal to about 1.5. The ratio of the weight percent glycerine content to the weight percent propylene glycol content of the vaporisable barrier may be greater than or equal to about 2. The ratio of the weight percent glycerine content to the weight percent propylene glycol content of the vaporisable barrier may be greater than or equal to about 2.5. The ratio of the weight percent glycerine content to the weight percent propylene glycol content of the vaporisable barrier may be greater than or equal to about 3.

The one or more plasticizers may comprise a combination of one or more carboxylic acids and one or more aerosol formers. The one or more plasticizers may comprise a combination of one or more carboxylic acids and one or more polyhydric alcohols. In some embodiments the vaporizable barrier comprises a combination of sorbitol, glycerine, propylene glycol and one or more carboxylic acids.

The vaporisable barrier may comprise water.

The vaporisable barrier may have a water content of greater than or equal to about 0.1 percent by weight. The vaporisable barrier may have a water content of greater than or equal to about 0.5 percent by weight. The vaporisable barrier may have a water content of greater than or equal to about 1 percent by weight. The vaporisable barrier may have a water content of greater than or equal to about 2 percent by weight. The vaporisable barrier may have a water content of greater than or equal to about 3 percent by weight. The vaporisable barrier may have a water content of greater than or equal to about 4 percent by weight. The vaporisable barrier may have a water content of greater than or equal to about 5 percent by weight. The vaporisable barrier may have a water content of greater than or equal to about 8 percent by weight. The vaporisable barrier may have a water content of greater than or equal to about 10 percent by weight. The vaporisable barrier may have a water content of greater than or equal to about 12 percent by weight. The vaporisable barrier may have a water content of greater than or equal to about 15 percent by weight.

The vaporisable barrier may have a water content of less than or equal to about 20 percent by weight. The vaporisable barrier may have a water content of less than or equal to about 15 percent by weight. The vaporisable barrier may have a water content of less than or equal to about 12 percent by weight. The vaporisable barrier may have a water content of less than or equal to about 10 percent by weight. The vaporisable barrier may have a water content of less than or equal to about 8 percent by weight. The vaporisable barrier may have a water content of less than or equal to about 5 percent by weight. The vaporisable barrier may have a water content of less than or equal to about 4 percent by weight. The vaporisable barrier may have a water content of less than or equal to about 3 percent by weight. The vaporisable barrier may have a water content of less than or equal to about 2 percent by weight. The vaporisable barrier may have a water content of less than or equal to about 1 percent by weight. The vaporisable barrier may have a water content of less than or equal to about 0.5 percent by weight. The vaporisable barrier may have a water content of less than or equal to about 0.1 percent by weight.

The vaporisable barrier may have a water content of between about 0.1 percent and about 20 percent by weight. The vaporisable barrier may have a water content of between about 0.1 percent and about 15 percent by weight. The vaporisable barrier may have a water content of between about 0.1 percent and about 12 percent by weight. The vaporisable barrier may have a water content of between about 0.1 percent and about 10 percent by weight. The vaporisable barrier may have a water content of between about 0.1 percent and about 8 percent by weight. The vaporisable barrier may have a water content of between about 0.1 percent and about 5 percent by weight. The vaporisable barrier may have a water content of between about 0.1 percent and about 4 percent by weight. The vaporisable barrier may have a water content of between about 0.1 percent and about 3 percent by weight. The vaporisable barrier may have a water content of between about 0.1 percent and about 2 percent by weight. The vaporisable barrier may have a water content of between about 0.1 percent and about 1 percent by weight. The vaporisable barrier may have a water content of between about 0.1 percent and about 0.5 percent by weight.

The vaporisable barrier may have a water content of between about 0.5 percent and about 15 percent by weight. The vaporisable barrier may have a water content of between about 1 percent and about 15 percent by weight. The vaporisable barrier may have a water content of between about 2 percent and about 15 percent by weight. The vaporisable barrier may have a water content of between about 3 percent and about 15 percent by weight. The vaporisable barrier may have a water content of between about 4 percent and about 15 percent by weight. The vaporisable barrier may have a water content of between about 5 percent and about 15 percent by weight. The vaporisable barrier may have a water content of between about 8 percent and about 15 percent by weight. The vaporisable barrier may have a water content of between about 10 percent and about 15 percent by weight. The vaporisable barrier may have a water content of between about 12 percent and about 15 percent by weight.

The vaporisable barrier may be formed from a formulation comprising water.

The formulation may have a water content of greater than or equal to about 40 percent by weight. The formulation may have a water content of greater than or equal to about 43 percent by weight. The formulation may have a water content of greater than or equal to about 45 percent by weight. The formulation may have a water content of greater than or equal to about 50 percent by weight. The formulation may have a water content of greater than or equal to about 55 percent by weight. The formulation may have a water content of greater than or equal to about 60 percent by weight. The formulation may have a water content of greater than or equal to about 65 percent by weight. The formulation may have a water content of greater than or equal to about 70 percent by weight. The formulation may have a water content of greater than or equal to about 75 percent by weight. The formulation may have a water content of greater than or equal to about 80 percent by weight. The formulation may have a water content of greater than or equal to about 85 percent by weight. The formulation may have a water content of greater than or equal to about 90 percent by weight. The formulation may have a water content of greater than or equal to about 95 percent by weight.

The formulation may have a water content of less than or equal to about 97 percent by weight. The formulation may have a water content of less than or equal to about 95 percent by weight. The formulation may have a water content of less than or equal to about 90 percent by weight. The formulation may have a water content of less than or equal to about 85 percent by weight. The formulation may have a water content of less than or equal to about 80 percent by weight. The formulation may have a water content of less than or equal to about 75 percent by weight. The formulation may have a water content of less than or equal to about 70 percent by weight. The formulation may have a water content of less than or equal to about 65 percent by weight. The formulation may have a water content of less than or equal to about 60 percent by weight. The formulation may have a water content of less than or equal to about 55 percent by weight. The formulation may have a water content of less than or equal to about 50 percent by weight. The formulation may have a water content of less than or equal to about 45 percent by weight.

The formulation may have a water content of between about 43 percent and about 97 percent by weight. The formulation may have a water content of between about 43 percent and about 95 percent by weight. The formulation may have a water content of between about 43 percent and about 90 percent by weight. The formulation may have a water content of between about 43 percent and about 85 percent by weight. The formulation may have a water content of between about 43 percent and about 80 percent by weight. The formulation may have a water content of between about 43 percent and about 75 percent by weight. The formulation may have a water content of between about 43 percent and about 70 percent by weight. The formulation may have a water content of between about 43 percent and about 65 percent by weight. The formulation may have a water content of between about 43 percent and about 60 percent by weight. The formulation may have a water content of between about 43 percent and about 55 percent by weight. The formulation may have a water content of between about 43 percent and about 50 percent by weight. The formulation may have a water content of between about 43 percent and about 45 percent by weight.

The formulation may have a water content of between about 45 percent and about 97 percent by weight. The formulation may have a water content of between about 50 percent and about 97 percent by weight. The formulation may have a water content of between about 45 percent and about 97 percent by weight. The formulation may have a water content of between about 50 percent and about 97 percent by weight. The formulation may have a water content of between about 55 percent and about 97 percent by weight. The formulation may have a water content of between about 60 percent and about 97 percent by weight. The formulation may have a water content of between about 65 percent and about 97 percent by weight. The formulation may have a water content of between about 70 percent and about 97 percent by weight. The formulation may have a water content of between about 75 percent and about 97 percent by weight. The formulation may have a water content of between about 80 percent and about 97 percent by weight. The formulation may have a water content of between about 85 percent and about 97 percent by weight. The formulation may have a water content of between about 90 percent and about 97 percent by weight. The formulation may have a water content of between about 95 percent and about 97 percent by weight.

The vaporisable barrier may be formed by drying the formulation.

The vaporisable barrier may be formed by providing a formulation comprising the components in the amounts discussed above and drying the formulation. The step of drying the formulation reduces the water content of the formulation to provide the barrier material having the component amounts discussed above.

The method of or forming the barrier may comprise combining the components of the barrier to provide a barrier formulation and subsequently drying the formulation to provide a barrier material. In some embodiments, the method may involve heating the components either before or after combining them.

Preferably, the components are heated to an elevated temperature of over 60 degrees Celsius, preferably over 70 degrees Celsius, preferably over 90 degrees Celsius.

Preferably, the formulation is maintained at the elevated temperature for a period of at least about 5 minutes, preferably at least about 10 minutes, preferably at least about 15 minutes, preferably at least about 20 minutes.

In some embodiments it may be advantageous to combine the components of the barrier/formulation is a specific order. In some embodiments the one or more plasticizers may be combined with the one or more polymers before adding any further components. Advantageously combining the components in this order may improve the processability of the formulation during production.

The step of drying the formulation may occur at an elevated temperature, the step of drying may occur at a temperature of at least 40 degrees Celsius. Alternatively, the step of drying the formulation may occur at ambient conditions or standard temperature and pressure.

As discussed below, the formulation may be dried and to form a barrier material and then the barrier applied to the storage portion or the formulation may be dried in-situ to provide the barrier.

The vaporisable barrier may be provided internally within the storage portion.

The vaporisable barrier may be attached to the storage portion. The vaporisable barrier may be attached to an internal surface of the storage portion.

The vaporisable barrier may be mechanically fixed to a component of the aerosol-generating article, the aerosol-generating device or the aerosol-generating system.

The vaporisable barrier may be mechanically fixed to a component of the aerosol-generating article, the aerosol-generating device or the aerosol-generating system by pressing the vaporisable barrier on to the component.

The vaporisable barrier may be mechanically fixed to a component of the aerosol-generating article, the aerosol-generating device or the aerosol-generating system by at least partially melting a portion of the vaporisable barrier and pressing the vaporisable barrier on to the component. Advantageously, partially melting a portion of the barrier prior to fixing the barrier to the component may improve adhesiveness. As disclosed below the barrier may be formed in situ by melt casting. This may advantageously increase adhesion between the barrier and the component.

The vaporisable barrier may be mechanically fixed to a liquid-side of a component of the aerosol-generating article, the aerosol-generating device or the aerosol-generating system.

The vaporisable barrier may be formed on a component of the aerosol-generating article, the aerosol-generating device or the aerosol-generating system. The vaporisable barrier may be formed on a liquid-side of a component of the aerosol-generating article, the aerosol-generating device or the aerosol-generating system.

The vaporisable barrier may be formed on a component of the aerosol-generating article, the aerosol-generating device or the aerosol-generating system by being cast onto the component. The vaporisable barrier may be formed on a component of the aerosol-generating article, the aerosol-generating device or the aerosol-generating system by being cast onto the component during assembly of the aerosol-generating article, the aerosol-generating device or the aerosol-generating system.

The component of the aerosol-generating article, the aerosol-generating device or the aerosol-generating system may be a heater. The component of the aerosol-generating article, the aerosol-generating device or the aerosol-generating system may be a transfer element. The component of the aerosol-generating article, the aerosol-generating device or the aerosol-generating system may be a wicking material. The component of the aerosol-generating article, the aerosol-generating device or the aerosol-generating system may be a mesh layer.

By liquid-side in reference to the location of the barrier in the present invention it is meant that the barrier is formed between the aerosol-forming substrate and the component of the aerosol forming article. It would also be appreciated that, in light of the nature of the barriers disclosed herein and the nature of the components of the aerosol-forming article, that the barrier may partially overlap with the component. For example, where the component is a transfer element the barrier may partially penetrate the transfer element structure. This may be particularly the case where the barrier is formed on the component. Advantageously, when the vaporisable barrier is formed on the liquid side of a component, it may be held in place by pressure exerted on it by the liquid.

The method may comprise providing the vaporisable barrier internally within the storage portion.

The method may comprise attaching the vaporisable barrier to the storage portion. The method may comprise attaching the vaporisable barrier to an internal surface of the storage portion.

The method may comprise casting the vaporisable barrier and then mechanically fixing the vaporisable barrier to a component of the aerosol-generating article, the aerosol-generating device or the aerosol-generating system.

The method may comprise mechanically fixing the vaporisable barrier to a component of the aerosol-generating article, the aerosol-generating device or the aerosol-generating system by pressing the vaporisable barrier on to the component.

The method may comprise mechanically fixing the vaporisable barrier to a component of the aerosol-generating article, the aerosol-generating device or the aerosol-generating system by at least partially melting a portion of the vaporisable barrier and then pressing the vaporisable barrier on to the component. Advantageously, partially melting a portion of the barrier prior to fixing the barrier to the component may improve adhesiveness. As disclosed below the barrier may be formed in situ by melt casting. This may advantageously increase adhesion between the barrier and the component.

The method may comprise casting the vaporisable barrier and then mechanically fixing to a liquid-side of a component of the aerosol-generating article, the aerosol-generating device or the aerosol-generating system.

The method may comprise solvent casting the vaporisable barrier and then drying the vaporisable barrier.

The method may comprise drying the vaporisable barrier at a temperature of greater than or equal to about 15 degrees Celsius. The method may comprise drying the vaporisable barrier at a temperature of greater than or equal to about 25 degrees Celsius. The method may comprise drying the vaporisable barrier at a temperature of greater than or equal to about 30 degrees Celsius. The method may comprise drying the vaporisable barrier at a temperature of greater than or equal to about 40 degrees Celsius. The method may comprise drying the vaporisable barrier at a temperature of greater than or equal to about 40 degrees Celsius.

The method may comprise drying the vaporisable barrier at a temperature of less than or equal to about 70 degrees Celsius. The method may comprise drying the vaporisable barrier at a temperature of less than or equal to about 60 degrees Celsius. The method may comprise drying the vaporisable barrier at a temperature of less than or equal to about 50 degrees Celsius. The method may comprise drying the vaporisable barrier at a temperature of less than or equal to about 40 degrees Celsius. The method may comprise drying the vaporisable barrier at a temperature of less than or equal to about 30 degrees Celsius.

The method may comprise drying the vaporisable barrier at a temperature of between about 15 degrees Celsius and about 70 degrees Celsius. The method may comprise drying the vaporisable barrier at a temperature of between about 25 degrees Celsius and about 60 degrees Celsius. The method may comprise drying the vaporisable barrier at a temperature of between about 35 degrees Celsius and about 50 degrees Celsius.

The method may comprise melt casting the vaporisable barrier.

The method may comprise forming the vaporisable barrier on a component of the aerosol-generating article, the aerosol-generating device or the aerosol-generating system. The method may comprise forming the vaporisable barrier on a liquid-side of a component of the aerosol-generating article, the aerosol-generating device or the aerosol-generating system. The vaporisable barrier may be formed in situ.

The method may comprise casting the vaporisable barrier on a component of the aerosol-generating article, the aerosol-generating device or the aerosol-generating system. The method may comprise casting the vaporisable barrier on a liquid-side of a component of the aerosol-generating article, the aerosol-generating device or the aerosol-generating system. The vaporisable barrier may be cast in situ.

The component of the aerosol-generating article, the aerosol-generating device or the aerosol-generating system may be a heater. The component of the aerosol-generating article, the aerosol-generating device or the aerosol-generating system may be a transfer element. The component of the aerosol-generating article, the aerosol-generating device or the aerosol-generating system may be a mesh layer.

The vaporisable barrier may be heat-treated after being formed.

The method may comprise heat treating the vaporisable barrier. The method may comprise heat treating the vaporisable barrier after it has been formed.

The vaporisable barrier may be heat treated at a temperature of greater than or equal to about 50 degrees Celsius. The vaporisable barrier may be heat treated at a temperature of greater than or equal to about 75 degrees Celsius. The vaporisable barrier may be heat treated at a temperature of greater than or equal to about 100 degrees Celsius. The vaporisable barrier may be heat treated at a temperature of greater than or equal to about 125 degrees Celsius. The vaporisable barrier may be heat treated at a temperature of greater than or equal to about 150 degrees Celsius.

The vaporisable barrier may be heat treated at a temperature of less than or equal to about 200 degrees Celsius. The vaporisable barrier may be heat treated at a temperature of less than or equal to about 175 degrees Celsius. The vaporisable barrier may be heat treated at a temperature of less than or equal to about 150 degrees Celsius. The vaporisable barrier may be heat treated at a temperature of less than or equal to about 125 degrees Celsius. The vaporisable barrier may be heat treated at a temperature of less than or equal to about 100 degrees Celsius.

The vaporisable barrier may be heat treated at a temperature of between about 75 degrees Celsius and 200 degrees Celsius. The vaporisable barrier may be heat treated at a temperature of between about 75 degrees Celsius and 175 degrees Celsius. The vaporisable barrier may be heat treated at a temperature of between about 75 degrees Celsius and 150 degrees Celsius. The vaporisable barrier may be heat treated at a temperature of between about 75 degrees Celsius and 125 degrees Celsius. The vaporisable barrier may be heat treated at a temperature of between about 75 degrees Celsius and 100 degrees Celsius.

The vaporisable barrier may be heat treated at a temperature of between about 50 degrees Celsius and 175 degrees Celsius. The vaporisable barrier may be heat treated at a temperature of between about 100 degrees Celsius and 175 degrees Celsius. The vaporisable barrier may be heat treated at a temperature of between about 125 degrees Celsius and 175 degrees Celsius. The vaporisable barrier may be heat treated at a temperature of between about 150 degrees Celsius and 175 degrees Celsius.

The vaporisable barrier may be heat treated in an oven. The vaporisable barrier may be heat treated in a ventilated oven.

Advantageously, heat treating the vaporisable barrier after it has been formed may decrease swelling of the vaporisable barrier. Heat treatment of the vaporisable barrier may improve crosslinking between the one or more polymers and the one or more plasticizers.

The storage portion may comprise a reservoir.

The heater may be an electric heater. The electric heater may comprise a resistive heating element. The electric heater may comprise an inductive heating element.

The heater may comprise a heating element. The heating element may be a grid element. The heating element may be a grid layer. The heating element may be a mesh element. The heating element may be a mesh layer. In such embodiments, the aerosol-forming substrate may flow into the interstitial spaces forming the grid or mesh.

For the avoidance of doubt, features described above in relation to the aerosol-generating article may also relate, where appropriate, to the aerosol-generating device and the aerosol-generating system, and *vice versa.*

Specific embodiments will now be described, by way of example only, with reference to the following examples and the accompanying drawings, in which:
Figure 1 shows schematically a sectional side view of an aerosol-generating system comprising an aerosol-generating device and an aerosol-generating article according to the invention;
Figure 2 shows schematically a sectional view of the aerosol-generating system of Figure 1, with the aerosol-generating article inserted into the aerosol-generating device;
Figure 3 shows schematically a sectional view of an alternative aerosol-generating system comprising an aerosol-generating device and an aerosol-generating article according to the invention;
Figure 4 shows schematically a sectional view of an aerosol-generating article according to the invention before it has been used; and
Figure 5 shows schematically a sectional view of an aerosol-generating article according to the invention after it has been used.

Aerosol-generating systems for delivering to a user typically comprise an atomiser configured to generate an inhalable aerosol from an aerosol-forming substrate. Some known aerosol-generating systems comprise a thermal atomiser such as an electric heater that is configured to heat and vaporise the aerosol-forming substrate to generate an aerosol. Other known aerosol-generating systems comprise a non-thermal atomiser that is configured to generate an aerosol from the aerosol-forming substrate using, for example, impinging jet, ultrasonic or vibrating mesh technologies. Typical aerosol-forming substrates for use in aerosol-generating systems are nicotine formulations, which may be liquid nicotine formulations comprising an aerosol former such as glycerine and/or propylene glycol.

An aerosol generating system can comprise an aerosol-generating device and an aerosol-generating article containing an aerosol-forming substrate. Typical aerosol-generating systems may suffer from a problem of unwanted leakage of the aerosol-forming substrate out of the aerosol-generating article. Leakage of an aerosol-forming substrate may occur in a number of different situations, such as: when there is too much of the aerosol-forming substrate in a reservoir of the aerosol-generating article; when the material forming one or more parts of the aerosol-generating article or system fails to retain the aerosol-forming substrate as designed; due to a change in pressure, for example when at a high altitude during transport by an aeroplane; or at a high temperature, for example due to hot weather.

It would be desirable to provide an aerosol-generating article that provides a reduced risk of leakage of the aerosol-forming substrate from the aerosol-generating article or system compared to typical aerosol-generating articles.

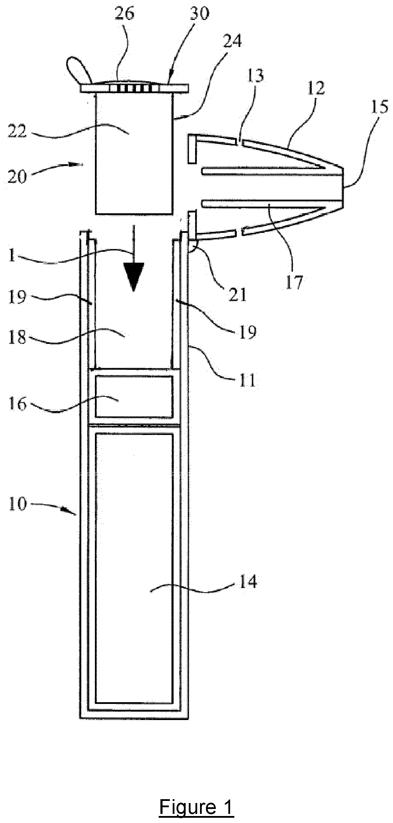

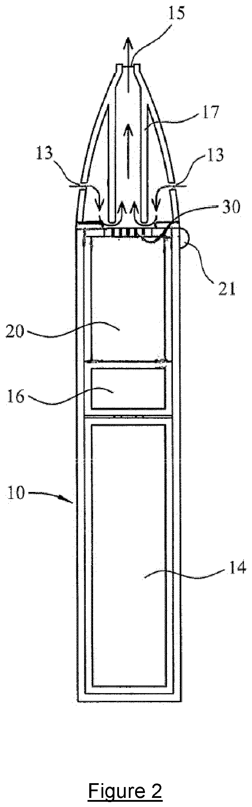

Figures 1 and 2 show an aerosol-generating system including an aerosol-generating device 10 and an aerosol-generating article 20. In this example, the aerosol-generating article 20 is cartridge.

The aerosol-generating device 10 is configured to receive the aerosol-generating article 20 in a cavity 18. The aerosol-generating article 20 includes a housing 24. The housing 24 defines a storage portion 22. The storage portion 22 has a storage portion opening that can be covered by a removable cover 26. An aerosol-forming substrate is disposed in the storage portion 22.

In the example shown in Figures 1 and 2, the aerosol-generating article 20 includes an atomiser configured to generate an aerosol from the aerosol-forming substrate in the storage portion 22. The atomiser may be a thermal atomiser. In the example shown in Figures 1 and 2 the atomiser is an electric heater 30. In other examples, the atomiser may be another type of atomiser, such as a non-thermal atomiser.

In the example of Figures 1 and 2, the aerosol-generating article 20 contains an aerosol-forming substrate and an atomiser and may therefore be referred to as a "cartomiser".

The aerosol-generating article 20 is replaceable by a user when the aerosol-forming substrate provided in the storage portion 22 is depleted.

Figure 1 shows the aerosol-generating article 20 just prior to insertion into the aerosol-generating device 10. The arrow 1 in Figure 1 indicates the direction of insertion of the aerosol-generating article 20 in to the aerosol-generating device 10.

The aerosol-generating device 10 is portable and has a size comparable to a conventional cigar or cigarette. The aerosol-generating device 10 comprises a main body 11 and a mouthpiece portion 12. The main body 11 contains a battery 14, such as a lithium iron phosphate battery, control electronics 16 and a cavity 18.

The mouthpiece portion 12 is connected to the main body 11 by a hinged connection 21 and can move between an open position as shown in Figure 1 and a closed position as shown in Figure 2. The mouthpiece portion 12 is placed in the open position to allow for insertion and removal of an aerosol-generating article 20 and is placed in the closed position when the aerosol-generating system is to be used to generate aerosol.

The mouthpiece portion 12 comprises a plurality of air inlets 13 and an outlet 15. In use, a user sucks or puffs on the outlet 15 to draw air from the air inlets 13, through the mouthpiece portion to the outlet 15, and thereafter into the mouth or lungs of the user. Internal baffles 17 are provided to force the air flowing through the mouthpiece portion 12 past the aerosol-generating article 20.

The housing 24 includes a capillary material soaked in the aerosol-forming substrate. The capillary material in this example is positioned adjacent the electric heater 30.

The cavity 18 has a circular cross-section and is sized to receive a housing 24 of the aerosol-generating article 20. Electrical connectors 19 are provided at the sides of the cavity 18 to provide an electrical connection between the control electronics 16 and battery 14 and corresponding electrical contacts on the aerosol-generating article 20. This setup allows power to be supplied to the electric heater 30.

Figure 2 shows the aerosol-generating article 20 inserted into the cavity 18 of the aerosol-generating device 10. In this position, the electrical connectors 19 rest against the corresponding electrical contacts on the aerosol-generating article 20. The cover 26 has been fully removed and the mouthpiece portion 12 has been moved to a closed position.

The mouthpiece portion 12 is retained in the closed position by a clasp mechanism (not illustrated). It will be apparent to a person of ordinary skill in the art that other suitable mechanisms for retaining the mouthpiece in a closed position may be used, such as a snap fitting or a magnetic closure.

The mouthpiece portion 12 in a closed position retains the aerosol-generating article 20 in electrical contact with the electrical connectors 19 so that a good electrical connection is maintained in use, whatever the orientation of the aerosol-generating system is.

In use, when the aerosol-generating device 10 is activated by a user, the electric heater 30 aerosolises at least a portion of the aerosol-forming substrate in the storage portion 22. As a user sucks or puffs on the outlet 15, air flows through the air inlets 13 and over the electric heater 30 and the capillary material. The air flowing over the electric heater 30 and the capillary material entrains the volatized aerosol components from the vaporised aerosol-forming substrate. The air with entrained aerosol-forming substrate then flows out through the outlet 15 and to the user. This air flow regime is shown in Figure 2.

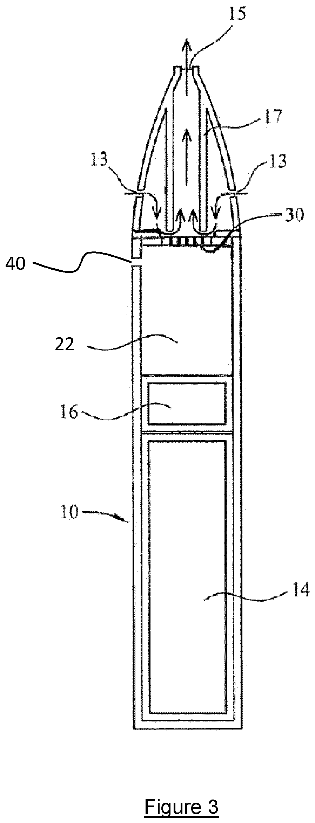

Figure 3 shows an alternative aerosol-generating system. The embodiment shown in Figure 3 works in much the same way as the embodiment shown in Figures 1 and 2. However, in the embodiment of Figure 3, the aerosol-generating article 20 is not removable from the aerosol-generating device 10. Instead, after the storage portion 22 has been depleted of aerosol-generating substrate, the storage portion 22 can be refilled by a user through a storage portion opening 40.

In Figure 3 the storage portion opening 40 is shown in an open position in which it can be refilled with aerosol-generating substrate. The storage portion opening 40 can however be sealed with a closure such as a cap (not shown).

The embodiment shown in Figure 3 otherwise works in a similar way to the aerosol-generating system shown in Figures 1 and 2.

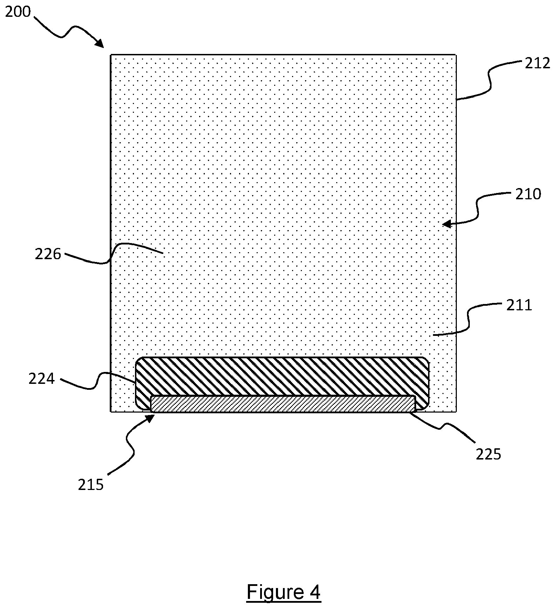

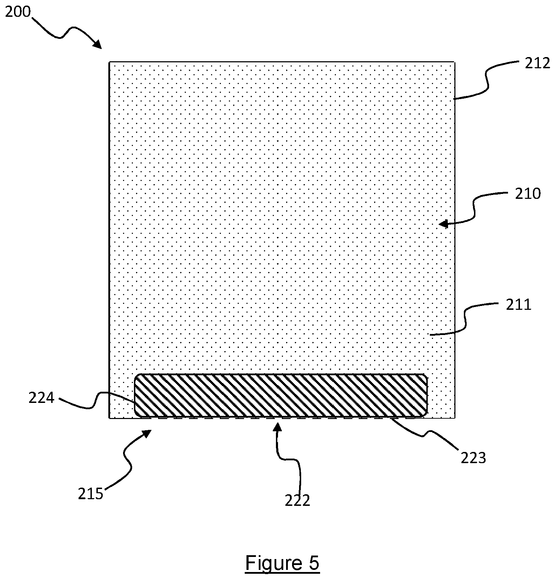

Figures 4 and 5 are schematic sectional views of an alternative aerosol-generating article 200. Figure 4 shows the aerosol-generating article 200 before it has been used by the user. The aerosol-generating article 200 includes a body 212 defining a storage portion 210 having a storage portion opening 215. Aerosol-forming substrate 211 is disposed in the storage portion 210. The aerosol-generating article 200 includes a heater 222 located across the storage portion opening 215. In this example, the heater 222 has a heating element in the form of a mesh layer 223. The aerosol-generating article 200 also includes a transfer element 224. The transfer element 224 is preferably formed from a porous material. In the example of Figure 4, the transfer element 224 is formed from a layer of glass fibers. The transfer element 224 provides control of the flow of the aerosol-forming substrate 211 from the storage portion 210 to the mesh layer 213 of the heater 222.

In use, the aerosol-forming substrate flows from the storage portion 210 and into the porous transfer element 224. The then flows to the mesh layer 223 of the heater 222, where it is thermally vaporised into an aerosol.

Figure 4 shows that the aerosol-generating article also includes a vaporisable barrier 225. The vaporisable barrier 225 is provided during manufacture of the aerosol-generating article 200. The vaporisable barrier 225 seals the storage portion 210 by blocking the flow of the aerosol-forming substrate 211 from the storage portion 210 to the heater 222.

In the example of Figure 4, the vaporisable barrier 225 is attached to the an internal surface of the storage portion 210. In this specific example, the vaporisable barrier 225 is attached to the heater 222, which forms a surface of the storage portion 210.

The vaporisable barrier 225 may be connected to the aerosol-generating article 200 through use of a number of different methods. In the example of Figure 4, the vaporisable barrier 225 has been mechanically fixed to the heater 222 after the vaporisable barrier 225 has been formed. In another example, the vaporisable barrier 225 may be formed on the aerosol-generating article 200 by casting a drop of a barrier formulation on the aerosol-generating article 200.

The vaporisable barrier 225 is formed so that it vaporises after the first use of the aerosol-generating article 200. That is, all of the mass of the vaporisable barrier 225 enters the vapour phase in the first heating cycle of the aerosol-generating article 200.

Figure 4 shows the aerosol-generating article 200 before first use and after it has been manufactured, with the vaporisable barrier 225 in place. Figure 5 shows the aerosol-generating article 200 after first use, during which the vaporisable barrier 225 has been vaporised by the heater 222.

Advantageously, the vaporisable barrier 225 that is applied to the aerosol-generating article 200 during manufacture of the aerosol-generating article 200 physically blocks flow of the aerosol-forming substrate 211 out of the storage portion 210 before the aerosol-generating article 200 has been first used. The vaporisable barrier 225 thereby prevents leakage of the aerosol-forming substrate 211 out of the aerosol-generating article 200 before it is first used, which may improve shelf-life of the aerosol-generating article 200. The vaporisable barrier 225 can even reduce or prevent leakage under pressure such as during transportation on an aeroplane.

In addition, given that the vaporisable barrier 225 is put in place during the manufacturing process, there is no need for a large window for a sealing tab, which gives more freedom for designing an aerosol-generating article 200. Not having a sealing tab also simplifies the assembly process.

The vaporisable barrier 225 is vaporised by the heater 222 during first use of the aerosol-generating article 200 (that is, during the first heating cycle). This allows the aerosol-generating article 200 to be used as normal by a user.

### Examples

Two mixtures for forming a vaporisable barrier according to the invention (Examples A and B) are prepared having the compositions shown in Table 1.

**Table 1**

| **Example** | | **A** | **B** |
|---|---|---|---|
| Glycerine (% by weight) | plasticizer | 28 | 17 |
| Propylene Glycol (% by weight) | plasticizer | 2.8 | 16 |
| Sodium Stearate (% by weight) | metal salt | 1.8 | 0 |
| Polyvinyl Alcohol (% by weight) | polymer | 7.2 | 7 |
| Water (% by weight) | | 60.2 | 60 |

The examples A and B are prepared by:
Mixing the polyvinyl alcohol, glycerine and propylene glycol together at 90 degrees Celsius;
Adding the sodium stearate to the mixture;
Keeping the formulation at 90 degrees Celsius for 20 minutes whilst stirring;
Drying the formulation at 40 degrees Celsius for a period of time in order to form the example; and
Applying the example to the aerosol-generating article 200.

The step of applying the example to the aerosol-generating article 200 depends on the method in which the vaporisable barrier 225 is being attached to or formed on the aerosol-generating article 200.

In the example of Figures 4 and 5, the vaporisable barrier 225 is mechanically fixed between the storage portion 210 and the heater 222. In this example, examples A and B are dried in for example a ventilated oven. The resulting film is then mechanically fixed to the aerosol-generating article 200 to provide the vaporisable barrier 225.

In another example, a drop of the example mixture is cast on to a component of the aerosol-generating article 200. The mixture then solidifies into the vaporisable barrier 225.

After formation of the vaporisable barrier 225, the storage portion 210 of the aerosol-generating article 200 can be filled with aerosol-forming substrate 211.

The exemplary embodiments described above are not intended to limit the scope of the claims. Other embodiments consistent with the exemplary embodiments described above will be apparent to those skilled in the art. Features described in relation to one embodiment may also be applicable to other embodiments. The scope of the present invention is defined by the appended claims.

## Claims

1. An aerosol-generating article (200) for use in an aerosol-generating system, the aerosol-generating article (200) comprising:
a storage portion (210) having an outlet;
an aerosol-forming substrate, the aerosol-forming substrate being contained within the storage portion (210); and
a vaporisable barrier (225) configured to seal the outlet,
wherein the vaporisable barrier (225) comprises one or more polymers, and
wherein the vaporisable barrier (225) has a polymer content of greater than or equal to 1 percent by weight,
wherein the vaporisable barrier (225) is configured to be vaporised during a first heating cycle of the aerosol-generating article (200) during first use of the aerosol-generating article (200) in the aerosol-generating system,
wherein the vaporisable barrier (225) comprises one or more metal salts, wherein the one or more metal salts are selected from the group consisting of metal alginates, metal benzoates, metal cinnamates, metal cycloheptanecarboxylates, metal levulinates, metal propanoates, metal stearates and metal undecanoates, preferably wherein the vaporisable barrier (225) comprises one or more metal stearates.

2. An aerosol-generating article (200) for use in an aerosol-generating system, the aerosol-generating article (200) comprising:
a storage portion (210);
an aerosol-forming substrate, the aerosol-forming substrate being contained within the storage portion (210);
a heater (222), the heater being configured to heat the aerosol-forming substrate; and
a vaporisable barrier (225) configured to seal the storage portion (210) by blocking the flow of the aerosol forming substrate from the storage portion (210) to the heater (222), the vaporisable barrier being provided between the storage portion and the heater,
wherein the vaporisable barrier (225) comprises one or more polymers, and
wherein the vaporisable barrier (225) has a polymer content of greater than or equal to 1 percent by weight,
wherein the vaporisable barrier (225) is configured to be vaporised during a first heating cycle of the aerosol-generating article (200) during first use of the aerosol-generating article (200) in the aerosol-generating system,
wherein the vaporisable barrier (225) comprises one or more metal salts, wherein the one or more metal salts are selected from the group consisting of metal alginates, metal benzoates, metal cinnamates, metal cycloheptanecarboxylates, metal levulinates, metal propanoates, metal stearates and metal undecanoates, preferably wherein the vaporisable barrier comprises one or more metal stearates.

3. An aerosol-generating article (200) according to claim 1 or claim 2, wherein the vaporisable barrier (225) is thermally degradable when heated to a temperature of between 60 degrees Celsius and 300 degrees Celsius.

4. An aerosol-generating article (200) according to claim 3, wherein the vaporisable barrier comprises a material that is vaporisable when heated to a temperature of between 180 degrees Celsius and 300 degrees Celsius.

5. An aerosol-generating article (200) according to claim 1 or claim 2, wherein the vaporisable barrier (225) has a polymer content of between about 5 percent by weight and about 40 percent by weight.

6. An aerosol-generating article (200) according to any of claims 1, 2 or 5, wherein the one or more polymers are selected from the group consisting of: polyvinyl acetate, polyvinyl alcohol, polyethylene glycol, polyglycolic acid, polylactic acid, polydioxanone, polycaprolactone, polyethylene, polypropylene glycol, low density polyethylene and starch.

7. An aerosol-generating article (200) according to any preceding claim, wherein the vaporisable barrier (225) comprises starch.

8. An aerosol-generating article (200) according to any preceding claim, wherein the vaporisable barrier (225) is mechanically fixed to a liquid-side of a component of the aerosol-generating article (200).

9. An aerosol-generating article according to any one of claims 1 to 7, wherein the vaporisable barrier (225) is formed on a component of the aerosol-generating article (200).

10. An aerosol-generating article (200) according to claim 8 or claim 9, wherein the component of the aerosol-generating article (200) is a heater (222) or a wicking material.

11. An aerosol-generating system comprising:
an aerosol-generating article (20) according to any of claims 1 to 10; and
an aerosol-generating device.

12. A method of manufacturing an aerosol-generating article (200), the method comprising:
providing an aerosol generating article (200) comprising: a storage portion (210); an aerosol-forming substrate, the aerosol-forming substrate being contained within the storage portion (210); and a heater (222), the heater being configured to heat the aerosol-forming substrate; and
providing a vaporisable barrier (225) between the storage portion (210) and the heater (222), the vaporisable barrier (225) being configured to seal the storage portion (210) by blocking the flow of the aerosol-forming substrate from the storage portion (210) to the heater (222),
wherein the vaporisable barrier (225) comprises one or more polymers, and
wherein the vaporisable barrier (225) has a polymer content of greater than or equal to 1 percent by weight,
wherein the vaporisable barrier (225) is configured to be vaporised during a first heating cycle of the aerosol-generating article (200) during first use of the aerosol-generating article (200) in the aerosol-generating system,
wherein the vaporisable barrier (225) comprises one or more metal salts, wherein the one or more metal salts are selected from the group consisting of metal alginates, metal benzoates, metal cinnamates, metal cycloheptanecarboxylates, metal levulinates, metal propanoates, metal stearates and metal undecanoates, preferably wherein the vaporisable barrier comprises one or more metal stearates.

## Patentansprüche

1. Aerosolerzeugender Artikel (200) zum Gebrauch in einem Aerosolerzeugungssystem, der aerosolerzeugende Artikel (200) umfassend:
einen Speicherabschnitt (210), aufweisend einen Auslass;
ein aerosolbildendes Substrat, wobei das aerosolbildende Substrat innerhalb des Speicherabschnitts (210) enthalten ist; und
eine zum Abdichten des Auslasses ausgelegte verdampfbare Sperre (225),
wobei die verdampfbare Sperre (225) ein oder mehrere Polymere umfasst, und
wobei die verdampfbare Sperre (225) einen Polymergehalt von mehr als oder gleich 1 Gewichtsprozent aufweist,
wobei die verdampfbare Sperre (225) ausgelegt ist, während eines ersten Erwärmungszyklus des aerosolerzeugenden Artikels (200) während des ersten Gebrauchs des aerosolerzeugenden Artikels (200) in dem Aerosolerzeugungssystem verdampft zu werden,
wobei die verdampfbare Sperre (225) ein oder mehrere Metallsalze aufweist, wobei das eine oder die mehreren Metallsalze ausgewählt sind aus der Gruppe bestehend aus Metallalginaten, Metallbenzoaten, Metallcinnamaten, Metallcycloheptancarboxylaten, Metalllevulinaten, Metallpropanoaten, Metallstearaten und Metallundecanoaten, bevorzugt wobei die verdampfbare Sperre (225) ein oder mehrere Metallstearate umfasst.

2. Aerosolerzeugender Artikel (200) zum Gebrauch in einem Aerosolerzeugungssystem, der aerosolerzeugende Artikel (200) umfassend:
einen Speicherabschnitt (210);
ein aerosolbildendes Substrat, wobei das aerosolbildende Substrat innerhalb des Speicherabschnitts (210) enthalten ist;
eine Heizvorrichtung (222), wobei die Heizvorrichtung zum Erwärmen des aerosolbildenden Substrats ausgelegt ist; und
eine verdampfbare Sperre (225), die zum Abdichten des Speicherabschnitts (210) durch Blockieren der Strömung des aerosolbildenden Substrats von dem Speicherabschnitt (210) zu der Heizvorrichtung (222) ausgelegt ist, wobei die verdampfbare Sperre zwischen dem Speicherabschnitt und der Heizvorrichtung vorgesehen ist,
wobei die verdampfbare Sperre (225) ein oder mehrere Polymere umfasst, und
wobei die verdampfbare Sperre (225) einen Polymergehalt von mehr als oder gleich 1 Gewichtsprozent aufweist,
wobei die verdampfbare Sperre (225) ausgelegt ist, während eines ersten Erwärmungszyklus des aerosolerzeugenden Artikels (200) während des ersten Gebrauchs des aerosolerzeugenden Artikels (200) in dem Aerosolerzeugungssystem verdampft zu werden,
wobei die verdampfbare Sperre (225) ein oder mehrere Metallsalze aufweist, wobei das eine oder die mehreren Metallsalze ausgewählt sind aus der Gruppe bestehend aus Metallalginaten, Metallbenzoaten, Metallcinnamaten, Metallcycloheptancarboxylaten, Metalllevulinaten, Metallpropanoaten, Metallstearaten und Metallundecanoaten, bevorzugt wobei die verdampfbare Sperre ein oder mehrere Metallstearate umfasst.

3. Aerosolerzeugender Artikel (200) nach Anspruch 1 oder Anspruch 2, wobei die verdampfbare Sperre (225) thermisch abbaubar ist, wenn sie auf eine Temperatur zwischen 60 Grad Celsius und 300 Grad Celsius erwärmt wird.

4. Aerosolerzeugender Artikel (200) nach Anspruch 3, wobei die verdampfbare Sperre ein Material umfasst, das bei Erwärmung auf eine Temperatur zwischen 180 Grad Celsius und 300 Celsius verdampfbar ist.

5. Aerosolerzeugender Artikel (200) nach Anspruch 1 oder Anspruch 2, wobei die verdampfbare Sperre (225) einen Polymergehalt zwischen etwa 5 Gewichtsprozent und etwa 40 Gewichtsprozent aufweist.

6. Aerosolerzeugender Artikel (200) nach einem der Ansprüche 1, 2 oder 5, wobei das eine oder die mehreren Polymere ausgewählt sind aus der Gruppe bestehend aus: Polyvinylacetat, Polyvinylalkohol, Polyethylenglykol, Polyglykolsäure, Polymilchsäure, Polydioxanon, Polycaprolacton, Polyethylen, Polypropylenglykol, niederdichtem Polyethylen und Stärke.

7. Aerosolerzeugender Artikel (200) nach einem beliebigen vorhergehenden Anspruch, wobei die verdampfbare Sperre (225) Stärke umfasst.

8. Aerosolerzeugender Artikel (200) nach einem beliebigen vorhergehenden Anspruch, wobei die verdampfbare Sperre (225) mechanisch an einer Flüssigkeitsseite einer Komponente des aerosolerzeugenden Artikels (200) befestigt ist.

9. Aerosolerzeugender Artikel nach einem der Ansprüche 1 bis 7, wobei die verdampfbare Sperre (225) auf einer Komponente des aerosolerzeugenden Artikels (200) gebildet ist.

10. Aerosolerzeugender Artikel (200) nach Anspruch 8 oder Anspruch 9, wobei die Komponente des aerosolerzeugenden Artikels (200) eine Heizvorrichtung (222) oder ein Dochtmaterial ist.

11. Aerosolerzeugungssystem, aufweisend:
einen aerosolerzeugenden Artikel (20) nach einem der Ansprüche 1 bis 10; und
eine Aerosolerzeugungsvorrichtung.

12. Verfahren zum Herstellen eines aerosolerzeugenden Artikels (200), das Verfahren umfassend:
Vorsehen eines aerosolerzeugenden Artikels (200), umfassend: einen Speicherabschnitt (210); ein aerosolbildendes Substrat, wobei das aerosolbildende Substrat innerhalb des Speicherabschnitts (210) enthalten ist; und eine Heizvorrichtung (222), wobei die Heizvorrichtung zum Erwärmen des aerosolerzeugenden Substrats ausgelegt ist; und
Vorsehen einer verdampfbaren Sperre (225) zwischen dem Speicherabschnitt (210) und der Heizvorrichtung (222), wobei die verdampfbare Sperre (225) zum Abdichten des Speicherabschnitts (210) durch Blockieren der Strömung des aerosolbildenden Substrats von dem Speicherabschnitt (210) zu der Heizvorrichtung (222) ausgelegt ist,
wobei die verdampfbare Sperre (225) ein oder mehrere Polymere umfasst, und
wobei die verdampfbare Sperre (225) einen Polymergehalt von mehr als oder gleich 1 Gewichtsprozent aufweist,
wobei die verdampfbare Sperre (225) ausgelegt ist, während eines ersten Erwärmungszyklus des aerosolerzeugenden Artikels (200) während des ersten Gebrauchs des aerosolerzeugenden Artikels (200) in dem Aerosolerzeugungssystem verdampft zu werden,
wobei die verdampfbare Sperre (225) ein oder mehrere Metallsalze aufweist, wobei das eine oder die mehreren Metallsalze ausgewählt sind aus der Gruppe bestehend aus Metallalginaten, Metallbenzoaten, Metallcinnamaten, Metallcycloheptancarboxylaten, Metalllevulinaten, Metallpropanoaten, Metallstearaten und Metallundecanoaten, bevorzugt wobei die verdampfbare Sperre ein oder mehrere Metallstearate umfasst.

## Revendications

1. Article de génération d'aérosol (200) pour une utilisation dans un système de génération d'aérosol, l'article de génération d'aérosol (200) comprenant :
une portion de stockage (210) ayant une sortie ;
un substrat formant aérosol, le substrat formant aérosol étant contenu au sein de la portion de stockage (210) ; et
une barrière vaporisable (225) configurée pour étanchéifier la sortie,
dans lequel la barrière vaporisable (225) comprend un ou plusieurs polymères, et
dans lequel la barrière vaporisable (225) a une teneur en polymère supérieure ou égale à 1 pour cent en poids,
dans lequel la barrière vaporisable (225) est configurée pour être vaporisée pendant un premier cycle de chauffage de l'article de génération d'aérosol (200) pendant la première utilisation de l'article de génération d'aérosol (200) dans le système de génération d'aérosol,
dans lequel la barrière vaporisable (225) comprend un ou plusieurs sels métalliques, dans lequel les un ou plusieurs sels métalliques sont choisis dans le groupe constitué par les alginates métalliques, les benzoates métalliques, les cinnamates métalliques, les cycloheptanecarboxylates métalliques, les lévulinates métalliques, les propanoates métalliques, les stéarates métalliques et les undécanoates métalliques, de préférence dans lequel la barrière vaporisable (225) comprend un ou plusieurs stéarates métalliques.

2. Article de génération d'aérosol (200) pour une utilisation dans un système de génération d'aérosol, l'article de génération d'aérosol (200) comprenant :
une portion de stockage (210) ;
un substrat formant aérosol, le substrat formant aérosol étant contenu au sein de la portion de stockage (210) ;
un dispositif de chauffage (222), le dispositif de chauffage étant configuré pour chauffer un substrat formant aérosol ; et une barrière vaporisable (225) configurée pour étanchéifier la portion de stockage (210) en bloquant l'écoulement du substrat formant aérosol depuis la portion de stockage (210) jusqu'au dispositif de chauffage (222), la barrière vaporisable étant prévue entre la portion de stockage et le dispositif de chauffage,
dans lequel la barrière vaporisable (225) comprend un ou plusieurs polymères, et
dans lequel la barrière vaporisable (225) a une teneur en polymère supérieure ou égale à 1 pour cent en poids,
dans lequel la barrière vaporisable (225) est configurée pour être vaporisée pendant un premier cycle de chauffage de l'article de génération d'aérosol (200) pendant la première utilisation de l'article de génération d'aérosol (200) dans le système de génération d'aérosol,
dans lequel la barrière vaporisable (225) comprend un ou plusieurs sels métalliques, dans lequel les un ou plusieurs sels métalliques sont choisis dans le groupe constitué par les alginates métalliques, les benzoates métalliques, les cinnamates métalliques, les cycloheptanecarboxylates métalliques, les lévulinates métalliques, les propanoates métalliques, les stéarates métalliques et les undécanoates métalliques, de préférence dans lequel la barrière vaporisable comprend un ou plusieurs stéarates métalliques.

3. Article de génération d'aérosol (200) selon la revendication 1 ou la revendication 2, dans lequel la barrière vaporisable (225) est thermiquement dégradable lorsqu'elle est chauffée à une température d'entre 60 degrés Celsius et 300 degrés Celsius.

4. Article de génération d'aérosol (200) selon la revendication 3, dans lequel la barrière vaporisable comprend une matière qui est vaporisable lorsqu'il est chauffé à une température d'entre 180 degrés Celsius et 300 degrés Celsius.

5. Article de génération d'aérosol (200) selon la revendication 1 ou la revendication 2, dans lequel la barrière vaporisable (225) a une teneur en polymère d'entre environ 5 pour cent en poids et environ 40 pour cent en poids.

6. Article de génération d'aérosol (200) selon l'une quelconque des revendications 1, 2 ou 5, dans lequel les un ou plusieurs polymères sont choisis dans le groupe constitué par : acétate de polyvinyle, alcool polyvinylique, polyéthylèneglycol, acide polyglycolique, acide polylactique, polydioxanone, polycaprolactone, polyéthylène, polypropylène glycol, polyéthylène basse densité et amidon.

7. Article de génération d'aérosol (200) selon l'une quelconque des revendications précédentes, dans lequel la barrière vaporisable (225) comprend de l'amidon.

8. Article de génération d'aérosol (200) selon l'une quelconque des revendications précédentes, dans lequel la barrière vaporisable (225) est attachée mécaniquement à un côté liquide d'un composant de l'article de génération d'aérosol (200).

9. Article de génération d'aérosol selon l'une quelconque des revendications 1 à 7, dans lequel la barrière vaporisable (225) est formée sur un composant de l'article de génération d'aérosol (200) .

10. Article de génération d'aérosol (200) selon la revendication 8 ou la revendication 9, dans lequel le composant de l'article de génération d'aérosol (200) est un dispositif de chauffage (222) ou une matière de mèche.

11. Système de génération d'aérosol comprenant :
un article de génération d'aérosol (20) selon l'une quelconque des revendications 1 à 10 ; et
un dispositif de génération d'aérosol.

12. Procédé de fabrication d'un article de génération d'aérosol (200), le procédé comprenant :
la fourniture d'un article de génération d'aérosol (200) comprenant : une portion de stockage (210) ; un substrat formant aérosol, le substrat formant aérosol étant contenu au sein de la portion de stockage (210) ; et un dispositif de chauffage (222), le dispositif de chauffage étant configuré pour chauffer le substrat formant aérosol ; et
la fourniture d'une barrière vaporisable (225) entre la portion de stockage (210) et le dispositif de chauffage (222), la barrière vaporisable (225) étant configurée pour étanchéifier la portion de stockage (210) en bloquant l'écoulement du substrat formant aérosol depuis la portion de stockage (210) vers le dispositif de chauffage (222),
dans lequel la barrière vaporisable (225) comprend un ou
plusieurs polymères, et
dans lequel la barrière vaporisable (225) a une teneur en polymère supérieure ou égale à 1 pour cent en poids,
dans lequel la barrière vaporisable (225) est configurée pour être vaporisée pendant un premier cycle de chauffage de l'article de génération d'aérosol (200) pendant la première utilisation de l'article de génération d'aérosol (200) dans le système de génération d'aérosol,
dans lequel la barrière vaporisable (225) comprend un ou plusieurs sels métalliques, dans lequel les un ou plusieurs sels métalliques sont choisis dans le groupe constitué par les alginates métalliques, les benzoates métalliques, les cinnamates métalliques, les cycloheptanecarboxylates métalliques, les lévulinates métalliques, les propanoates métalliques, les stéarates métalliques et les undécanoates métalliques, de préférence dans lequel la barrière vaporisable comprend un ou plusieurs stéarates métalliques.
